# NEW EUROPEAN PATENT SPECIFICATION

(11) **EP 1 836 970 B2**
(45) Date of publication and mention of the opposition decision: **13.03.2013**
(45) Mention of the grant of the patent: 16.12.2009
(21) Application number: 07251229.6
(22) Date of filing: 22.03.2007
(51) Int. Cl.: A61B 17/06, A61B 19/00

(54) **Marked suture**
Markiertes Wundnahtmaterial
Suture marquée

(30) Priority: 23.03.2006 US 277283
(43) Date of publication of application: 26.09.2007
(73) Proprietor: ETHICON ENDO-SURGERY, INC., Cincinnati, Ohio 45242 (US)
(72) Inventor: Zwolinski, Andrew M., Cincinnati Ohio 45226 (US); Ortiz, Mark S., Milford Ohio 45150 (US); Stokes, Michael J., Cincinnati Ohio 45244 (US)
(74) Representative: Tunstall, Christopher Stephen

(56) References cited:
- GB-A- 2 273 566
- US-A- 3 840 015
- US-A- 3 949 755
- US-A- 4 127 109
- US-A- 5 401 257
- US-A- 2003 050 667
- US-A1- 2002 193 811
- US-A1- 2002 193 811
- US-A1- 2003 025 023
- US-A1- 2003 025 023
- US-A1- 2005 240 267
- US-A1- 2005 277 983
- US-B2- 6 562 024
- US-B2- 6 626 925

## Description

### FIELD OF THE INVENTION

The present invention relates broadly to an improved suture material, in particular, a suture material that includes at least one marking.

### BACKGROUND OF THE INVENTION

It is well known that suture material is commonly used to repair openings in skin, internal organs, blood vessels, and a variety of other tissues of the human body.
Conventionally, suturing of human tissue occurs during open surgery or minimally invasive surgical procedures.

In certain minimally invasive surgical procedures, e.g., endoscopic and laparoscopic surgeries, a surgeon performs diagnostic and therapeutic procedures at the surgical site through a natural body aperture or through one or more small incisions, using instruments specially designed for this purpose. Problems encountered by a surgeon in such minimally invasive surgical procedures include reduced visibility and field of vision, as well as potential orientation difficulties when performing the required manipulations at the surgical site, such as suturing tissue.

Suturing procedures can be particularly challenging in minimally invasive surgical procedures. For example, it can be difficult for a surgeon to determine various properties of the suture material being used, such as the amount of suture used, direction of the suture, and the forces to which the suture is subjected. As a result, use of such suture material adds to the visibility and orientation problems encountered by a surgeon when suturing in a minimally invasive surgical procedure.

US 2005/277983 discloses apparatuses and methods for optimizing the force for securing anchors against tissue. More particularly, this document relates to apparatus and methods for optimizing the force for loading basket-type anchors within or against tissue within a body.

US 2003/025023 discloses a dispenser for suture, including a suture reel on which the suture is mounted and an indicator that the suture is soon to be depleted. The indicator can he a visual marking on the suture, or it can be an audible and/or vibratory effect obtained by the dispenser housing interacting with the suture through one or more view ports or by interaction of a follower cam with the reel structure as the suture becomes depleted.

US 2002/193811 discloses a suture passer comprising a longitudinally extending hollow cannula having a central passage slidingly receivable of a surgical suture; a manually graspable handle connected to the hollow cannula for manipulation thereof, the handle having an upper surface; first guide means, connected to the upper surface of the handle, proximate a proximal end of the handle for releasably, guidingly, holding the surgical suture; second guide means, connected to the upper surface of the handle, distal to the first guide means, for releasably, guidingly, holding the surgical suture.

Accordingly, a need exists for an improved suture material that provides enhanced ease of use, particularly in minimally invasive surgical procedures.

### SUMMARY OF THE INVENTION

The present invention generally provides an improved suture material according to claim 1 for enhanced ease of use, particularly in minimally invasive surgical procedures. The suture material can include a flexible strand of suture that can have formed thereon at least one marking. The marking can provide information on the various properties of the suture, such as indicating a at least one use characteristic of the suture. Examples of suture use characteristics include length of suture and depth of suture penetration.

A marking on the suture can include a plurality of graduations. The graduations can be formed around at least a portion of a circumference of the suture and be spaced from each other along a length of the suture. The graduations are spaced at increasing increments along a length of the suture. Such graduations can provide a qualitative or quantitative indication of the progression, or depth of penetration, of the suture through the material being sutured. The increase or decrease in the spacing of the graduations can also be indicative of direction.

An exemplary method for suturing with suture material according to the aspects of the present invention is also discussed. The method includes the steps of providing a length of suture having formed thereon at least one marking indicative of a use characteristic of the suture, and passing the suture through tissue that is to be sutured while observing a use characteristic of the suture.

### BRIEF DESCRIPTION OF THE DRAWINGS

The invention will be more fully understood from the following detailed description taken in conjunction with the accompanying drawings, in which:

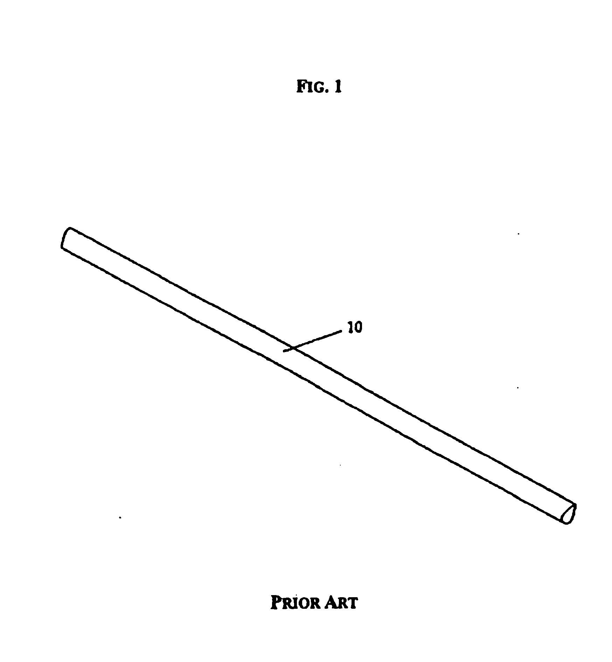

FIG. 1 presents a perspective view of a prior art strand of suture material;

FIG. 2 presents a perspective view of a strand of suture having formed thereon markings that are comprised of graduations spaced at increasing increments along the length of the suture;

### DETAILED DESCRIPTION OF THE INVENTION

Certain exemplary embodiments will now be described to provide an overall understanding of the principles, structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides an improved suture material that includes a flexible strand of suture that can have formed thereon at least one marking. The marking is adapted to provide various informational properties of the suture to a user of the improved suture material. For example, a marking can indicate information relating to at least one use characteristic of the suture. Examples of use characteristics include: the progression of the suture and the depth of penetration of the suture. In use, the improved suture material is passed through tissue that is being sutured, and a user is able to discern at least one use characteristic of the suture based on the indications provided by at least one marking that is formed on the suture. The improved suture material is advantageous in that it provides an enhanced ease of use of a suture, and facilitates in alleviating some of the visibility and orientation problems encountered when suturing tissue, particularly in a minimally invasive surgical procedure.

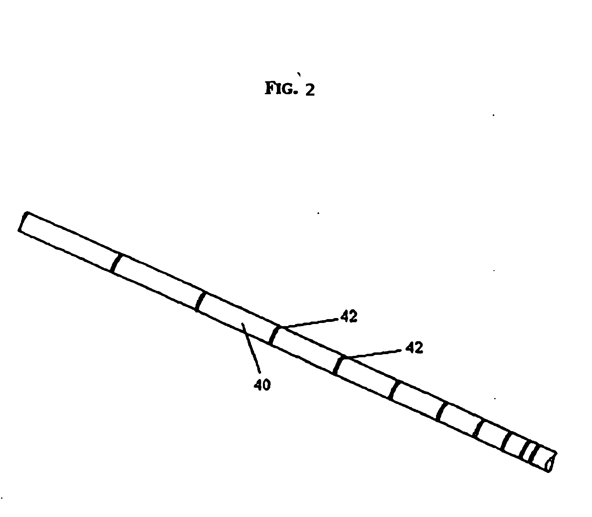

FIG. 2 shows an embodiment of the improved suture material having formed thereon a plurality of graduations 42. The graduations 42 can be formed around at least a portion of the circumference of a suture 40. Graduations 42 can also be formed around the entire portion of the circumference of suture 40, in a continuous manner or in a broken manner. The graduations 42 can be spaced at increasing increments along the length of suture 40. For example, the graduations can be rather closely spaced (e.g., about 1 mm) at a proximal end of the suture and have increasingly greater spacing intervals moving distally along the suture. One skilled in the art will appreciate that the thickness of the graduations 42 can vary.

Graduations 42 can be indicative of a use characteristic of suture 40 by providing a user with qualitative information or a quantitative value. For example, the increasing increments of the graduations 42 can provide qualitative information or a quantitative value that is indicative of the length of progression of suture 40 through tissue being sutured. In a further example, graduations 42 can provide a qualitative or quantitative indication of the depth of penetration by suture 40 through tissue being sutured. In another example, graduations 42 can provide an indication of direction based on whether the incremental spacing of graduations 42 is increasing or decreasing.

One skilled in the art will appreciate that the suture material of the present invention can be otherwise constructed to indicate other use characteristics of the suture material and/or to enhance visibility of the suture. For example, at least one marking formed on a flexible strand of suture as described herein can be radiopaque. Alternatively, the markings on a suture described herein can be of a contrasting color. For example, at least one marking can be of a color other than the color of the suture. Further, the markings formed on the sutures described herein can be of a texture different from a texture of the suture. For example, the markings can be indented in the strand of suture, or raised on the suture strand. In addition, a person skilled in the art will appreciate that the improved suture material of the present invention can include any combination of markings or features described herein, as well as other markings or features known in the art.

The suture material of the present invention can be used in a variety of surgical procedures, for example, for repairing or re-attaching or torn incised tissue. In an exemplary embodiment, the suture material can be used in a suturing procedure conducted during a minimally invasive surgical procedure, such as an endoscopic or laparoscopic procedure. In another exemplary embodiment, the suture material is used in the context of stitching epidermal disruptions. It will be understood, however, that the exemplary method described herein is equally applicable to connecting detached tissue in other contexts as well, such as during open and invasive surgical procedures.

An exemplary method for suturing with the suture material described herein includes providing a length of suture that can have formed thereon at least one marking which can be indicative of a use characteristic of the suture.

The improved suture material of the present invention can be formed from a variety of known materials that are suitable for use in forming suture material. For example, the suture can be made from a variety of polymers that can be resorbable or non-resorbable.

The embodiments of the present invention can be formed onto the suture material in a variety of processes. By way of example, the various suture markings embodying the present invention can be applied to the sutures described herein by an off-set printing process. The markings can also be applied to the sutures of the present invention by a pad printing process, which can involve obtaining an ink from a master, and subsequently transferring the impression to the suture material.

The invention is not to be limited by what has been particularly shown and described, except as indicated by the appended claim.

## Claims

1. A suture material comprising:
a flexible strand of suture (40) having formed thereon at least one marking indicative of a use characteristic of the suture;
**characterised in that** the at least one marking a plurality of graduations (42) and wherein the graduations are spaced at increasing increments along a length of the suture indicative of the use characteristics of length of suture progression or depth of suture penetration.

## Patentansprüche

1. Nahtmaterial, das aufweist:
einen flexiblen Nähfaden (40), auf dem wenigstens eine Markierung gebildet ist, die für eine Nutzungseigenschaft des Nähfadens bezeichnend ist;
**dadurch gekennzeichnet, dass** die wenigstens eine Markierung eine Vielzahl von Abstufungen (42) ist, wobei die Abstufungen mit zunehmenden Inkrementen über eine Länge des Nähfadens beabstandet sind, so dass sie für die Nutzungseigenschaft der Länge des Nähfortschritts oder der Tiefe der Fadeneindringung bezeichnend sind.

## Revendications

1. Matériau de suture comprenant :
un brin flexible de suture (40) sur lequel est formé au moins un marquage indicatif d'une caractéristique d'utilisation de la suture ;
**caractérisé en ce que** le au moins un marquage est une pluralité de graduations (42), les graduations étant espacées à des incréments croissants suivant une longueur de la suture indicatifs des caractéristiques d'utilisation de la longueur de progression de la suture ou de la profondeur de pénétration de la suture.
